# EUROPÄISCHE PATENTSCHRIFT

(11) **EP 1 523 239 B1**
(45) Veröffentlichungstag und Bekanntmachung des Hinweises auf die Patenterteilung: **26.11.2008**
(21) Anmeldenummer: 03729777.7
(22) Anmeldetag: 24.06.2003
(51) Int. Cl.: A01N 59/08, A01N 59/00, A61K 33/20, A61L 2/16, A61L 2/23, A61K 8/20

(54) **VERWENDUNG EINER ZUSAMMENSETZUNG UND EINER DIE ZUSAMMENSETZUNG ENTHALTENDEN REINIGUNGSTABLETTE ZUR DESINFEKTION**
USE OF A COMPOSITION AND A CLEANING TABLET CONTAINING SAID COMPOSITION FOR DISINFECTING PURPOSES
UTILISATION D'UNE COMPOSITION ET D'UN COMPRIME DE NETTOYAGE CONTENANT CETTE COMPOSITION A DES FINS DE DESINFECTION

(30) Priorität: 24.06.2002 CH 107902; 24.03.2003 CH 509032003
(43) Veröffentlichungstag der Anmeldung: 20.04.2005
(73) Patentinhaber: Bonyf AG, 9490 Vaduz (LI)
(72) Erfinder: WOLLWAGE, Peter, FL-9493 Mauren (LI)
(74) Vertreter: Hasler, Erich
(86) Internationale Anmeldenummer: PCT/CH2003/000421
(87) Internationale Veröffentlichungsnummer: WO 2004/000025

(56) Entgegenhaltungen:
- EP-A- 0 265 709
- EP-A- 0 451 105
- WO-A-01/07035
- WO-A-01/67864
- WO-A-87/05187
- WO-A-91/03936
- WO-A-91/07876
- WO-A-97/19708
- US-A- 3 577 532

## Beschreibung

### Technisches Gebiet der Erfindung

Die vorliegende Erfindung betrifft die Verwendung einer Zusammensetzung und eine die Zusammensetzung enthaltende Reinigungstablette zur Desinfektion, insbesondere von dentalen Gegenständen und zur topischen Behandlung von mit Mikroorganismen, nämlich Candida oder Retro- oder Herpesviren befallenen Körperstellen.

### Stand der Technik

Es ist bekannt, dass Zahnbürsten Bakterien, Virus und andere krankmachende Organismen beherbergen können. Zahnbürsten können auch mit Keimen kontaminiert werden, welche in Badezimmern unvermeidbar vorhanden sind. Jedes mal, wenn die Toilette gespült wird, werden gefährliche Keime in die Luft befördert. Solche Keime können auch Zahnbürsten kontaminieren, welche oft in Zahnbechern mit der Bürste nach oben aufbewahrt werden

Zahnbürsten sind verschmutzt, wenn sie nur für ein paar wenige Tage gebraucht wurden. Es konnte nachgewiesen werden, dass Bakterien, Viren, und andere Keime leicht 24 Stunden überleben können. Da mehrere Zahnbürsten oft in nächster Nähe voneinander aufbewahrt werden, besteht auch ein Risiko der Kreuz-Kontaminierung. Krankheitsmachende Bakterien und Viren können deshalb die Zahnbürsten von anderen Familienmitgliedern kontaminieren. Folglich sollten Zahnbürsten ziemlich oft und auf jeden Fall nach einer Krankheit gewechselt werden.

Aus den oben angeführten Gründen wurde bereits vorgeschlagen, Zahnbürsten von Zeit zu Zeit in Geschirrspülern zu reinigen. Dies ist ein einfacher Weg, um Zahnbürsten zu Hause zu sterilisieren. Auf Reisen jedoch ist normalerweise kein Geschirrspüler zur Verfügung.

EP-A-0 081 962 offenbart eine selbstauflösende Tablette zur Reinigung von künstlichen Gebissen. Die Tablette enthält eine Kombination einer Sauerstoff freisetzenden Perborat-Verbindung und einer Hypochlorit freisetzenden Chloroisocyanurat-Verbindung, wenn die Tablette mit Wasser in Kontakt kommt. Die Tablette enthält auch alkalische Verbindungen, welche für einen pH-Wert von ungefähr 11 sorgen, wenn die Tablette im wässerigen Medium aufgelöst wird.

Die US 3,936,385 offenbart ebenfalls eine selbstauflösende Reinigungstablette enthaltend eine - in Kontakt mit Wasser - Sauerstoff freisetzende Peroxo-Verbindung und eine - in Kontakt mit Wasser - Hypochlorit freisetzende Chlorverbindung, wobei das Verhältnis von Hypochlorit und Sauerstoff wenigstens 1.1 : 1 beträgt. Im Weiteren enthält die Tablette eine alkalische Verbindung in einer solchen Menge, dass ein pH-Wert von > 10.5 resultiert.

Als Nachteil der vorerwähnten Tabletten gilt deren Chlorgeruch, welcher beim Auflösen dieser Hypochlorit freisetzenden Tablette auftritt. Die EP-A-0164 472 schlägt deshalb vor, der Zusammensetzung einen Hypochlorit-Deaktivator beizugeben. Als Deaktivatoren werden beispielsweise Natriumperborat Monohydrat und Natriumnitrit eingesetzt, welche mit dem Hypochlorit zu reagieren vermögen. Der Deaktivator wird mit Verzögerung freigesetzt, sodass das Hypochlorit zunächst seine Wirkung entfalten kann. Als eine Lösung zur verzögerten Freisetzung des Deaktivators wird vorgeschlagen, dass die Tablette zwei Bereiche mit unterschiedlichen Zusammensetzungen und Lösungseigenschaften aufweist. Dabei enthält der erste Bereich der Tablette die Reinigungszusammensetzung, und der zweite, sich langsamer auflösende Bereich enthält den Deaktivator. Auch bei diesen Tabletten ist der pH-Wert der Reinigungslösung mindestens 7.5 und vorzugsweise jedoch zwischen 8.5 und 11. Eine ähnliche Lösung beschreibt auch die EP-A-0 360 299. Die vorgenannten Tabletten sind aufwändig herzustellen und daher teuer.

Die EP-A-0 451105 schlägt zur Verbesserung der Reinigungswirkung vor, eine aus zwei Teilen bestehende Reinigungstablette mit unterschiedlichen Zusammensetzungen einzusetzen, bei welcher die Tablettenteile oder -bereiche nicht übereinander, sondern nebeneinander angeordnet sind. Dies hat den Vorteil, dass sich die Tablette unabhängig von Ihrer Lage immer gleich schnell auflöst. Für den Fall, dass eine pH-Änderung während des Auflösens der Tablette erwünscht ist, schlägt die EP-A-0 451105 vor, eine saure Verbindung dem einen Teil der Tablette und eine basische (neutralisierende) Verbindung dem anderen Teil der Tablette beizumengen. Die saure Komponente kann dabei anfänglich für einen pH-Wert der Reinigungslösung von weniger als 2.5 sorgen. Durch vollständige und verzögerte Auflösung der basischen Verbindung wird der pH-Wert allerdings dann auf mindestens 5.5 oder darüber angehoben, d.h. die insgesamt in der Tablette enthaltenen sauren Komponenten liegen im Extremfall im Vergleich zu den basischen Bestandteilen nur im geringen Überschuss, im Normalfall jedoch - wie dies bei den bekannten Reinigungstabletten der Fall ist - im Unterschuss vor. Hauptbestandteile der Tablette der EP-A-0 451105 können saure und basische Verbindungen, Oxidationsmittel, Bleichmittel, Chelatbildner, oberflächenaktive Stoffe, Schmiermittel und Additive sein. Als Bleichmittel werden u.a. Hypochlorit bildende Stoffe oder Stoffkominbationen vorgeschlagen, welche in Kontakt mit Wasser Hypochlorit freisetzen oder bilden. Als Beispiel für eine solche Stoffkombination wird unter anderem auch eine Mischung aus Natriumchlorid und Natriumpersulfat genannt. Es ist allerdings kein erklärtes Ziel der EP-A-0 451105 aus Natriumchlorid und Natriumpersulfat in situ Chlor zu generieren. Ausserdem ist bekannt, dass zur Bildung von Hypochlorit ein alkalisches Milieu vorliegen sollte. Die EP-A-0 451105 macht auch keine Angaben zur spezifischen Wirksamkeit der beispielhaft vorgeschlagenen Zusammensetzungen.

Gemäss der EP-A-0 451 105 kann eine mehrteilige Tablette mit Bereichen unterschiedlicher Zusammensetzungen eine Vielzahl unterschiedlicher Stoffe enthalten, wobei jedoch in der Beschreibung nur ein konkretes Ausführungsbeispiel wiedergegeben ist. Diese beispielhafte Zusammensetzung enthält keine salzförmigen Chlorid- oder Bromidverbindungen in Kombination mit einem starken Oxidationsmittel und einer Säure resp. einem Überschuss Säure bei gleichzeitiger Anwesenheit von basischen Stoffen.

Aus der WO 97/19708 ist bekannt, dass Chlor freisetzende Verbindungen eine bakterizide Wirkung haben. Solche Verbindungen werden seit langem dazu benutzt, um beispielsweise Kindergeschirr zu reinigen und zu desinfizieren. Die WO 97/19708 schlägt eine verbesserte Zusammensetzung vor, welche eine gute Reinigungs- und Desinfektionswirkung besitzt. Die Zusammensetzung enthält ein anionisches Detergenz, einen Phoshat-Builder, Borax, ein Alkalimetallchlorid oder -sulfat, sowie eine Chlor freisetzende Verbindung. Gemäss WO 97/19708 trägt der Zusatz eines Alkalimetallchlorids dazu bei, die Langzeitstabilität der Chlor freisetzenden Verbindung zu erhöhen. Die Zusammensetzung der WO 97/19708 erwies sich als wirksam zur Abtötung von Cholibakterien und Staphylokokken.

Die WO 91/03936 offenbart eine antivirale Zusammensetzung enthaltend 0.01 bis 5 Teile eines anorganischen Chlorids, 25 bis 60 Teile eines Oxidationsmittels, 3 bis 8 Teile Sulphaminsäure, bis 20 Teile einer nicht-reduzierend wirkenden organischen Säure und 10 bis 30 Teile eines Alkalimetall-Phosphats. In einer wässerigen Lösung entwickelt die Zusammensetzung einen pH-Wert zwishcen 1.2 und 5.5. Als Oxidationsmittel werden vorzugsweise Peroxyphtalat oder Kaliumpersulfat eingesetzt. Die Zusammensetzung ist wirksam gegen verschiedene Virenarten.

Die WO 87/05187 offenbart ebenfalls eine trockene, wasserlösliche biozide Zusammensetzung mit einem breiten Wirkungsspektrum gegen verschiedenste Virenarten. Die Zusammensetzung enthält ein Alkalimetallphosphat, eine oberflächenaktiver Stoff, ein starkes Oxidationsmittel wie Kaliumpersulfat, Sulfaminsäure, eine nicht-reduzierend wirkende organische Säure und weniger als 50% einer anorganischen Chloridverbindung. Die Zusammensetzung ist als Desinfektionsmittel für den Einsatz auf Farmbetrieben gedacht und wirkt auch gegen verschiedene Bakterienstämme.

Die WO 91/ 07876 offenbart ein Desinfektionsmittel für Sanitärartikel, medizinische Instrumente etc. welche gegen Retroviren, insbesondere HIV-Viren wirksam ist. Die Zusammensetzung enthält organische Chlorverbindungen wie z.B. Chloramin-T.

Die WO 01/67864 offenbart ebenfalls eine Zusammensetzung enthaltend Kaliumpersulfat, Natriumchlorid, Sulfaminsäure, Apfelsäure, Dinatriumpyrophosphat und einem oberflächenaktiven Stoff für die pharmazeutische Verwendung. Als mögliche Verwendungen sind die prophylaktische Behandlung von Hepatitis und HIV Infektionen oder die Beschichtung von chirurgischen oder dentalen Gegenständen genannt. Es wird kein spezifischer pH-Wert offenbart.

Es ist bekannt, dass der Fungus Candida albicans schmerzhafte Stellen im Gaumen und Rachenbereich hervorrufen kann. Bislang wurde jedoch in der Mundhygiene diesem Aspekt wenig Beachtung geschenkt. Untersuchungen haben ausserdem gezeigt, dass auf herausnehmbaren Zahnprothesen eine Zahl von anderen krankmachenden Mikroorganismen vorkommen. Gefunden wurde beispielsweise gram-negative Bakterien, welche zu endotoxischer Sepsis und systemischen Erkrankungen führen können.

Obwohl die bekannten, selbstauflösenden und im Handel erhältlichen Reinigungstabletten angeblich eine desinfizierende Reinigungswirkung haben, lässt deren fungizide Wirkung, insbesondere gegen Candida, sehr zu wünschen übrig. Versuche haben gezeigt, dass herkömmliche Tabletten nicht in der Lage sind, Candida wirksam zu reduzieren. Auch ist von bekannten Reinigungstabletten nicht bekannt, dass Retro- oder Herpesviren unschädlich gemacht werden.

### Aufgabe der Erfindung

Es ist Aufgabe der vorliegenden Erfindung, ein Mittel zur Reinigung und Desinfektion von künstlichen Gebissen, Zahnbürsten und von mit Mikroorganismen wie Candida, Retro- oder Herpesviren befallenen Gegenständen und Körperstellen vorzuschlagen. Ein weiteres Ziel ist es, Mittel bereitzustellen, welche einfach gehandhabt, gut auf Reisen mitgenommen und wenig Platz beanspruchen. Noch ein Ziel ist es, Mittel bereitzustellen, mit welchen Bakterien wie Streptococcen und Pseudomonas, Pilze wie der Candida albicans, sowie Viren, insbesondere Herpesviren wirksam unschädlich gemacht werden können. Noch ein Ziel ist es, eine pharmazeutisch wirksame Zusammensetzung zum Unschädlichmachen von Candida, insbesondere des Fungus Candida albicans, vorzuschlagen.

### Beschreibung der Erfindung

Gegenstand der vorliegenden Erfindung ist die neuartige Verwendung einer Zusammensetzung enthaltend
- wenigstens eine salzförmige Chloridverbindung in Form eines Alkali- oder Erdalkalimetallsalzes und wenigstens ein geeignetes Oxidationsmittel zur in situ Produktion von Chlor bei Auflösung der Zusammensetzung in wässeriger Lösung, wobei das Oxidationspotential des Oxidationsmittels in wässeriger Lösung mindestens im beanspruchten pH-Bereich höher als das Oxidationspotential von Cl¹⁻/Cl^{o} ist. ,
- eine Säure in einer solchen Menge, dass die in einer bestimmten Menge einer wässerigen Lösung aufgelöste Zusammensetzung einen pH-Wert < 6, vorzugsweise < 5, 5 und ganz besonders bevorzugt < 5,0 erzeugt ist, sowie
- gegebenenfalls einen oberflächenaktiven Stoff (Tensid) oder Stoffgemisch, Aromastoffe, Hilfsstoffe und Bindemittel,
zur Desinfektion von mit Candida befallenen dentalen Gegenständen, wie von Zahnbürsten, Gebissen und dergleichen. Zur Überraschung des Erfinders kann mit Chlor Candida unschädlich gemacht werden. Das Ergebnis ist insofern überraschend, als bekannte in Form von selbstauflösenden Reinigungstabletten vorliegende Zusammensetzungen Candida nicht abzutöten vermögen.

Gemäss einer besonders vorteilhaften Ausführungsvariante enthält die Stoffkombination eine salzförmige Chloridverbindung und wenigstens ein geeignetes Oxidationsmittel zur in situ Produktion von Chlor bei Auflösung der Zusammensetzung in wässeriger Lösung. Diese Stoffkombination ist besonders wirksam, weil Chlor in situ gebildet werden.

Vorteilhaft ist in der Zusammensetzung eine Säure enthalten. Es hat sich zur Überraschung des Erfinders gezeigt, dass im sauren Milieu, d.h. bei einem pH-Wert < 7, die Wirksamkeit der Zusammensetzung deutlich besser ist.

Zweckmässigerweise ist die Säure in einer solchen Menge in der Zusammensetzung enthalten ist, dass die in einer bestimmten Menge einer wässerigen Lösung vollständig aufgelöste Zusammensetzung einen pH-Wert < 6, vorzugsweise < 5,5 und ganz besonders bevorzugt < 5,0 erzeugt. Eine solche Zusammensetzung hat den Vorteil, dass auch Staphylokokken, Streptokokken und Pseudomonas, innert sehr kurzer Zeit unschädlich gemacht werden. Zur Überraschung des Erfinders hat sich gezeigt, dass im sauren Milieu eine deutlich bessere keimtötende Wirkung - sowohl hinsichtlich des Wirkungsspektrums als auch hinsichtlich der Effektivität - erreicht werden kann als bei einem pH-Wert > 7, wie dies bei konventionellen, seit langem bekannten Zusammensetzungen der Fall ist. Insbesondere ist es möglich, bei einem pH-Wert < 5,5 auch den Fungus candida albicans innert weniger Minuten praktisch vollständig abzutöten. Die Zusammensetzung kann als Feststoffgemisch oder aber als wässerige Zusammensetzung vorliegen.

Das Chloratom kann in der Ausgangsverbindung entweder in Form eines Alkali- oder Erdalkalimetallsalzes oder einer anderen, in wässeriger Lösung Chloridionen freisetzenden Verbindung vorliegen, oder an einen organischen Rest gebunden sein. Chemische Verbindungen, welche Chlor abspalten können, sind beispielsweise Chloramin B (N-Chlorbenzolsulfonamido-Natrium), Chloramin T (p-Toluolsulfonchloramin-Natrium), Dichloramin T (p-Toluolsulfondichloramid), Halazon (p-Dichlorsulfamylbenzoesäure), Dichlorcyanursäure, Trichlorcyanursäure, TCM (Trichlormelamin), 1,3-Dichlor-5,5-dimethylhydantoin, Dichlorglycoluril, Succinchlorimid oder Chloroazodin (N,N'-dichlorodiazodicarbonamidin). Diese Verbindungen können allein oder in einer beliebigen Mischung eingesetzt werden. Die vorerwähnten Verbindungen können im sauren Milieu eine antivirale und fungizide Wirkung entfalten.

Vorteilhaft ist die Chlor generierende Stoffkombination eine Chlorverbindung in Form eines Alkali, Erdalkali- oder anderen Metallsalzes und ein geeignetes Oxidationsmittel. In Verbindung mit wenigstens einem geeigneten Oxidationsmittel kann durch Reaktion mit den in Lösung vorhandenen Chloridionen (Cl-) Chlor (Cl₂) oder Dichloroxid (Cl₂O⁻) gebildet werden. Zur in situ Chlorproduktion können unterschiedliche, dem Fachmann bekannte Zusammensetzungen resp. Stoffkombinationen eingesetzt werden. Der Vorteil einer derartigen Zusammensetzung ist, dass das Chlor relativ rasch freigesetzt wird und daher eine schnelle Wirkung eintritt. Es versteht sich von selbst, dass das Oxidationspotential des Oxidationsmittels in Lösung mindestens beim bevorzugten pH-Bereich < 6, resp. < 5,5 höher als das Oxidationspotential von Cl¹⁻/Cl^{o} sein soll, damit die erwünschte Reaktion eintreten kann.

Vorteilhaft enthält die Zusammensetzung weitere keimtötende Verbindungen, wie z.B. Kaliummonopersulfat, Kalium-Caroate, Natriumperoxycarbonat, Natriumbenzoat, Subtilisin, Kaliumbenzoat, Chlorhexidin, eine Kombination von Chlorhexidin und Thymol, Cetylpyridiniumchlorid, Halogen abspaltende Verbindungen wie PVP-Jod und Cyanursäurechlorid, und/oder Formaldehyd abspaltende Verbindungen wie Paraformaldehyd und/ oder Methylolverbindungen etc.

Gemäss einer bevorzugten Ausführungsform ist die Zusammensetzung ein Feststoffgemisch. Um die Auflösung und Durchmischung der Lösung zu verstärken, enthält die Zusammensetzung zweckmässigerweise Brausesalze, d.h. Mittel zur Lösungsbeschleunigung, welche im Englischen auch "effervescents" genannt werden. Die Mittel zur Lösungsbeschlemigung sind beispielsweise eine Carbonat (CO₃²⁻) oder Bicarbonat (H CO₃⁻) enthaltende Verbindung, wie Natriumcarbonat oder Natriumbicarbonat, und eine Säure. Als Säuren können beispielsweise Carbonsäuren, insbesondere Dicarbonsäuren, oder auch jede andere dem Fachmann bekannte Säure eingesetzt werden, welche vorzugsweise physiologisch unbedenklich ist. Vorzugsweise werden solche Säuren eingesetzt, welche in der Natur gut abbaubar sind.

Obwohl grundsätzlich unterschiedliche Oxidationsmittel eingesetzt werden können, ist in der Zusammensetzung als Oxidationsmittel vorteilhaft eine Hydrogenperoxosulfatverbindung oder Persulfatverbindung oder Wasserstoffperoxid enthalten. Eine vorteilhafte Zusammensetzung, welche kostengünstig herstellbar ist, enthält Kaliumhydrogenmonopersulfat (KHSO₅), Kochsalz (NaCl) und eine Säure, vorzugsweise in Form einer Mono-, Di- oder Tricarbonsäure wie z.B. Oxalsäure, Weinsäure, Bernsteinsäure oder Zitronensäure. Es ist vorzugsweise eine solche Menge Säure in der Zusammensetzung enthalten, dass nach dem Auflösen der Zusammensetzung im wässerigen Milieu ein pH-Wert < 5, 5 vorzugsweise < 5 erreicht ist. Zitronensäure ist physiologisch völlig unbedenklich, wird in der Natur rasch abgebaut und daher bevorzugt in der Zusammensetzung eingesetzt.

Eine besonders gute Desinfektions- und Reinigungswirkung erhält man dann, wenn die Zusammensetzung zusätzlich wenigstens ein geeignetes Tensid enthält. Dabei ist darauf zu achten, dass das eingesetzte Tensid mit dem Desinfektionsmittel kompatibel ist. Grundsätzlich können unterschiedliche, dem Fachmann bekannte Tenside eingesetzt werden. Es ist jedoch von Bedeutung, dass die Tenside bei einem pH-Wert kleiner 8, vorzugsweise kleiner 7, stabil sind. Unter "stabil" soll in diesem Zusammenhang verstanden werden, dass max. 10% des eingesetzten Tensids in Lösung bei Raumtemperatur innerhalb von 30 Minuten zerfallen. In Verbindung mit der erfindungsgemässen Zusammensetzung ist bevorzugt, dass das eingesetzte Tensid bei pH 6 bis 4, vorzugsweise bei einem pH < 5.5, nicht ausgefällt oder zersetzt wird. Vorzugsweise werden als Tenside Fettalkohol-Polyglykoläther, Alkylbenzolsulfonate, Alkylsulfonate eingesetzt. Besonders bewährt haben sich anionische Tenside, vorzugsweise aus der Gruppe der Alkylethersulfate, wie Fettalkoholethersulfat-Alkalisalze, z.B. Natrium-n-alkyl-C₁₂₋₁₄-diglyloläthersulfat Fettsäureamidopropyl Betain und/oder Natriumlaurylsulfosuccinat oder Natrium-n-alkyl-C₁₂₋₁₄-diglykoläthersulfat. Weiter eignen sich beispielsweise Na-Laurylsulfat, Na-Laurylsulfoacetatund Trinatriumphosphat. Vorzugsweise werden in der Zusammensetzung bei Raumtemperatur als Festkörper (Pulver) vorliegende Tenside eingesetzt.

Vorteilhaft ist die Zusammensetzung in Tabletten- oder Granulatform bereitgestellt. Tabletten oder Granulate können gut portioniert und auf Reisen mitgenommen werden. Die Zusammensetzung kann so dosiert sein, dass beim Auflösen der Tablette oder einer Packung Granulat in einem Glas Wasser eine wirksame, keimabtötende Mischung entsteht. Zweckmässigerweise enthält die Zusammensetzung ein Bindemittel, und optional Aroma-, Farb- und Hilfsstoffe wie Stoffe zur Wasserenthärtung, Füllstoffe und dergleichen. Als Bindemittel kann beispielsweise modifizierte Maisstärke, mikrokristalline Cellulose, Sorbito, hydrierte Soyatriglyceride, Polyethylenglykol wie PEG 180, PEG 150, PEG 75, Polyvinylpyrolidone, ein Copolymer von Ethylenoxid und Propylenoxid, Polyvinylpyrolidon oder ein Copolymer von Polyvinylpyrolidon Vinylacetat oder Zuckerderivate wie Laktose oder Kombinationen aus den genannten Verbindungen eingesetzt werden. Der gewichtsmässige Anteil des Bindemittels beträgt zweckmässigerweise maximal ungefähr 30% und liegt vorzugsweise zwischen 5 und 25%.

Zweckmässigerweise beträgt der gewichtsmässige Anteil der die Lösungsgeschwindigkeit beschleunigenden Komponenten im Bereich zwischen 15 und 40%, vorzugsweise 15 bis 30% des Gesamtgewichts der Tablette. Vorteilhaft übertrifft die eingesetzte Menge des Tensids (Gewichtsprozente) die eingesetzte Menge des Bakterizids (Gewichtsprozente).

Die erfindungsgemässe Zusammensetzung findet Verwendung zur Desinfektion von dentalen Gegenständen, insbesondere von Zahnbürsten und Gebissen, von Rasiergeräten und dergleichen, sowie zur Körperpflege, wie Fuss- und Handbäder. Von Bedeutung ist insbesondere, dass Candida, insbesondere der Candida albicans, welcher oft Ursache für Entzündungen der Mundhöhle ist, abgetötet werden kann. Candida finden sich oft auf Zahnprothesen und gelegentlich Zahnbürsten, sodass deren Bekämpfung für die menschliche Hygiene von besonderer Bedeutung ist. Von Bedeutung ist insbesondere auch die pharmazeutische Verwendung der Zusammensetzung, beispielsweise zur Herstellung eins Mittels zur Behandlung von mit Candida befallenen Körperstellen.

Gegenstand der Erfindung ist auch die Verwendung der Zusammensetzung zur Herstellung eines Mittels zur Desinfektion von mit Candida befallenen oder behafteten Körperteilen, insbesondere von Schleimhäuten der Mundhöhle. Die Zusammensetzung hat sich als wirksam erwiesen bei der Bekämpfung von Herpesviren, insbesondere Human herpesvirus 1(HHV-1; herpes simplex virus), family Herpesviridae, subfamily alphaherpesviridae, Human herpesvirus 5(HHV-5) Human cytomegalovirus strain Toledo, family Herpesviridae, subfamily betaherpesvirinae, aber auch von Retroviren wie der Family Retroviridae (Spumavirus genus).

Die Zusammensetzung eignet sich insbesondere aber auch zur Bekämpfung von mit Staphylokokken, insbesondere Staphylococcus aureus, Streptococcus pyogenes, Streptococcus mutans, Escherichia coli, Pseudomonas aeruginosa, Simian Foamy Virus (SFV-bab) strain OCOM1-26, befallenen Körperstellen oder Gegenständen.

### Beispiel:

Eine beispielhafte Zusammensetzung enthält folgende Komponenten:

| Stoff | in Gew.-prozenten (%) |
|---|---|
| Kaliumhydrogen-Monopersulfat | 5 |
| Na-Laurylsulfat | 15 |
| Natrium-bicarbonat | 20 |
| Zitronensäue | 30 |
| Bindemittel | 20 |
| Natriumchlorid | 10 |

Die Wirkung der erfindungsgemässen Zusammensetzungen wurde anhand folgender Mischung getestet:

| | |
|---|---|
| Streptococcus pyogenes ATCC 19615 | |
| Streptococcus mutans ATCC 25175 | |
| Pseudomonas aeruginosa | |
| Candida albicans | |
| Escherichia coli ATCC 25922 | RT |
| Anfangskonzentration: | 10⁵ - 10⁶ CFU/ml |

Die oben genannten Mikroorganismen wurden auf jeweils 2 Zahnbürsten angesiedelt, und diese dann in eine Testlösung getaucht. Die Testlösung wurde hergestellt, indem eine halbe Tablette in 100 ml Wasser bei einer Temperatur von 21 °C aufgelöst wurde. Eine ganze Tablette wog 2.5 g, die halbe Tablette demzufolge 1.25 g. Es stellte sich heraus, dass die Bakterien-/ Pilzmischung bereits nach 5 Minuten abgetötet war. Die Endkonzentration der Mikroorganismen betrug jeweils < 10¹.

In einer anderen Versuchsreihe wurden mehrere Stückchen einer Zahnprothese (Methylmethacrylat) in eine Nährlösung gegeben, welche mit Test-Mikroorganismen inokuliert wurde. In der Nährlösung ergab sich eine Anfangskonzentration von ca.10⁶ Mikroorganismen/Milliliter. Anschliessend wurde die Nährlösung während insgesamt ca. 12 Stunden bei erhöhter Temperatur geschüttelt und gleichzeitig belüftet. Nach den ersten 6 Stunden wurde die Lösung dekantiert und frische Nährlösung zugegeben. Die Flüssigkeit wurde danach abgegossen, und die Prothesenstücke in Petrischalen getrocknet.

Die Prothesenstücke wurden danach in 2 Gruppen geteilt, wobei die erste Gruppe während 5 Minuten in steriles Wasser gelegt, und die zweite Gruppe in eine erfindungsgemässe Zusammensetzung gelegt wurde. Danach wurde die Wirksamkeit überprüft, indem die Zahl der Mikroorganismen auf der Protesenoberfläche bestimmt wurde.

Eine hohe keimabtötende Wirkung konnte insbesondere bei den folgenden Mikroorganismen festgestellt:
Candida, insbesondere Candida albicans, Herpesviren, Staphylokokken, insbesondere Staphylococcus aureus, gram-negative Bakterien wie Pseudomonas aeruginosa, Herpesviren, insbesondere Human herpesvirus 1(HHV-1; herpes simplex virus), family Herpesviridae, subfamily alphaherpesviridae, Human herpesvirus 5(HHV-5) Human cytomegalovirus strain Toledo, family Herpesviridae, subfamily betaherpesvirinae, Simian Foamy Virus (SFV-bab), Retroviren wie Family Retroviridae (Spumavirus genus).

Zur Desinfektion wird eine mit Mikroorganismen behaftete Zahnprothese eine bestimmte Zeit, typischerweise eine bis fünf Minuten, einem Chlor oder Chloroxid resp. Brom oder Bromoxid freisetzenden Stoffgemisch ausgesetzt. Dazu wird eine selbstauflösende Reinigungstablette zusammen mit der zu desinfizierenden Zahnprothese in ein leeres gelegt und dieses anschliessend mit Wasser gefüllt. Beim Auflösen der Tablette wird die wässerige Lösung durchmischt. Gleichzeitig wird Chlor resp. Brom freigesetzt oder in situ gebildet. Durch die Agitation der Lösung gelangt Chlor resp. Brom an alle Stellen, sodass ein gute desinfizierende Wirkung erzielt wird.

Die Erfindung betrifft die Verwendung einer Zusammensetzung enthaltend
- wenigstens eine salzförmige Chloridverbindung in Form eines Alkali- oder Erdalkalimetallsalzes und wenigstens ein geeignetes Oxidationsmittel zur in situ Produktion von Chlor bei Auflösung der Zusammensetzung in wässeriger Lösung, wobei das Oxidationspotential des Oxidationsmittels in wässeriger Lösung mindestens im beanspruchten pH-Bereich höher als das Oxidationspotential von Cl1-/Clo ist. ,
- eine Säure in einer solchen Menge, dass die in einer bestimmten Menge einer wässerigen Lösung aufgelöste Zusammensetzung einen pH-Wert < 6, vorzugsweise < 5, 5 und ganz besonders bevorzugt < 5,0 erzeugt ist,, sowie
- gegebenenfalls einen oberflächenaktiven Stoff (Tensid) oder Stoffgemisch, Aromastoffe, Hilfsstoffe und Bindemittel, zur Herstellung eines pharmazeutisch wirksamen Mittels zur Behandlung von mit Candida albicans befallenen oder behafteten Körperteilen.

## Patentansprüche

1. Verwendung einer Zusammensetzung enthaltend
- wenigstens eine salzförmige Chloridverbindung in Form eines Alkali- oder Erdalkalimetallsalzes und wenigstens ein geeignetes Oxidationsmittel zur in situ Produktion von Chlor bei Auflösung der Zusammensetzung in wässeriger Lösung, wobei das Oxidationspotential des Oxidationsmittels in wässeriger Lösung mindestens im beanspruchten pH-Bereich höher als das Oxidationspotential von Cl¹-/Cl^{o} ist. ,
- eine Säure in einer solchen Menge, dass die in einer bestimmten Menge einer wässerigen Lösung aufgelöste Zusammensetzung einen pH-Wert < 6, vorzugsweise < 5, 5 und ganz besonders bevorzugt < 5,0 erzeugt ist, sowie
- gegebenenfalls einen oberflächenaktiven Stoff (Tensid) oder Stoffgemisch, Aromastoffe, Hilfsstoffe und Bindemittel,
zur Desinfektion von mit Candida befallenen dentalen Gegenständen, wie von Zahnbürsten und Gebissen.

2. Verwendung nach Anspruch 1, **dadurch gekennzeichnet, dass** die Zusammensetzung in Form eines Feststoffgemisches, insbesondere eines Granulates oder einer Tablette, vorliegt.

3. Verwendung nachAnspruch 1 oder 2, **dadurch gekennzeichnet, dass** die Zusammensetzung Mittel zur Lösungsbeschleunigung (Brausesalze = Effervescent), beispielsweise eine Carbonat- oder Bicarbonat enthaltende Verbindung wie z.B. Natriumcarbonat oder Natriumbicarbonat, und eine mindestens stöchiometrische Menge Säure zur Erzeugung eines pH-Wertes < 7 enthält.

4. Verwendung nach einem der Ansprüche 1 bis 3, **dadurch gekennzeichnet, dass** als Oxidationsmittel eine Hydrogenperoxosulfatverbindung enthalten ist.

5. Verwendung nach einem der Ansprüche 1 bis 4, **dadurch gekennzeichnet, dass** die Zusammensetzung mindestens Kaliumhydrogenmonopersulfat (KHSO₅) als Oxidationsmittel, eine salzförmige Chloridverbindung, z.B. Kochsalz (NaCl), als Chloridverbindung und eine Carbonsäure, vorzugsweise eine Mono-, Di oder Tricarbonsäure, wie Wein- oder Zitronensäure, zur Erzeugung eines sauren pH-Werts bei aufgelöster Zusammenseizung, enthält.

6. Verwendung nach einem der Ansprüche 1 bis 5, **dadurch gekennzeichnet, dass** die Zusammensetzung ein Bindemittel, und optional Aroma-, Farb- und Hilfsstoffe wie Stoffe zur Wasserenthärtung, Füllstoffe und dergleichen, enthält, und in Tabletten oder Granulatform vorliegt

7. Verwendung nach einem der Ansprüche 1 bis 6, **dadurch gekennzeichnet, dass** die Zusammensetzung wenigstens ein Tensid (oberflächenaktiver Stoff oder Stoffgemisch) enthält.

8. Verwendung nach einem der Ansprüche 1 bis 7, **dadurch gekennzeichnet, dass** die Zusammensetzung in Form einer selbstauflösenden Reinigungstablette vorliegt.

9. Verwendung nach Anspruch 8, **dadurch gekennzeichnet, dass** die Zusammensetzung oder die Reinigungstablette eine homogene Zusammensetzung hat.

10. Verwendung nach Anspruch 8 oder 9, **dadurch gekennzeichnet, dass** die Zusammensetzung oder die Reinigungstablette in wässeriger Lösung, vorzugsweise in Wasser, in Gegenwart des zu desinfizierenden Gegenstands oder Körperteils aufgelöst wird.

11. Verwendung nach einem der Ansprüche 1 bis 10, **dadurch gekennzeichnet, dass** das Bindemittel, ein Copolymer von Ethylenoxid und Propylenoxid, Polyvinylpyrolidon oder ein Copolymer von Polyvinylpyrolidon und Vinylacetat ist.

12. Verwendung einer Zusammensetzung enthaltend
- wenigstens eine salzförmige Chloridverbindung in Form eines Alkali- oder Erdalkalimetallsalzes und wenigstens ein geeignetes Oxidationsmittel zur in situ Produktion von Chlor bei Auflösung der Zusammensetzung in wässeriger Lösung, wobei das Oxidationspotential des Oxidationsmittels in wässeriger Lösung mindestens im beanspruchten pH-Bereich höher als das Oxidationspotential von Cl¹⁻/Cl^{o} ist.,
- eine Säure in einer solchen Menge, dass die in einer bestimmten Menge einer wässerigen Lösung aufgelöste Zusammensetzung einen pH-Wert < 6, vorzugsweise < 5, 5 und ganz besonders bevorzugt < 5,0 erzeugt ist" sowie
- gegebenenfalls einen oberflächenaktiven Stoff (Tensid) oder Stoffgemisch, Aromastoffe, Hilfsstoffe und Bindemittel, zur Herstellung eines pharmazeutisch wirksamen Mittels zur Behandlung von mit Candida albicans befallenen oder behafteten Körperteilen.

## Claims

1. Use of a composition containing
- at least one saline chloride compound in the form of a salt of an alkali or an alkaline earth metal and at least one suitable oxidation means for the in situ production of chlorine during the dissolution of the composition in an aqueous solution, where the oxidation potential of the oxidation means in an aqueous solution is higher than the oxidation potential of Cl1/Cl⁰ at least in the claimed range,
- an acid in such quantity that the composition dissolved in a certain quantity of an aqueous solution will produce a pH-value of less than 6, preferably less than 5.5 and even more preferably less than 5, as well as
- whenever appropriate, a surface-active substance (tenside) or substance mixture, aromatic substances, adjuvants and binders,
for the desinfection of dental objects such as toothbrushes, dentures and the like contaminated by candida.

2. Use in accordance with Claim 1, **characterized in that** the composition is available in the form of a mixture of solids, especially a granulate or a tablet.

3. Use in accordance with Claim 1 or Claim 2, **characterized in that** the composition contains means for accelerating the dissolution (effervescent salts - effervescents), for example a compound containing a carbonate or a bicarbonate such as, for example sodium carbonate or sodium bicarbonate and at least a stoichiometric quantity of acid for the production of a pH-value of less than 7 is contained in the composition.

4. Use in accordance with any one of Claims 1 to 3, **characterized in that** a hydrogen peroxosulphate compound is contained as oxidation means.

5. Use in accordance with any one of Claims 1 to 4, **characterized in that** the composition contains at least potassium hydrogen peroxosulphate as oxidizing agent, a saline chlorine compound, e.g. cooking salt (NaCl) as chloride compound and a carbon acid, preferably a mono-, di- or tricarbon acid, such as tartaric or citric acid, for the production of an acid pH-value in the dissolved composition.

6. Use in accordance with any one of Claims 1 to 5, **characterized in that** the composition contains a binder and, optionally, aromatic and colouring substances and adjuvants such as substances for softening the water, fillers and the like, and is available in tablets or granulate form.

7. Use in accordance with Claims 1 to 6, **characterized in that** the composition contains at least one tensid (surface-active substance or substance mixture).

8. Use in accordance with any one of Claims 1 to 7, **characterized in that** the composition is available in the form a self-dissolving cleaning tablet.

9. Use in accordance with Claim 8, **characterized in that** the composition or the cleaning tablet has a homogeneous composition

10. Use in accordance with any one of Claims 8 or 9, **characterized in that** the composition or the cleaning tablet is dissolved in an aqueous solution, preferable in water, in the presence of the object or body part that is to be disinfected.

11. Use in accordance with any one of Claims 1 to 10, **characterized in that** the binder is a copolymer of ethylene oxide and propylene oxide, polyvinyl pyrolidon or a copolymer of polyvinyl pyrolidon and vinyl acetate.

12. Use of a composition containing
- at least one saline chloride compound in the form of salt of an alkaline of alkali earth metal and at least one suitable oxidizing agent for the in situ production of chlorine during the dissolution of the composition in an aqueous solution, where the oxidation potential of the oxidizing agent in aqueous solution is greater than the oxidation potential of Cl¹⁻/Cl⁰ at least within the claimed pH-range,
- an acid such quantity that the composition dissolved in a certain quantity of an aqueous solution produces a pH-value of less than 6, preferably less than 5.5 and even more preferably less than 5.0, as well as,
- whenever appropriate, a surface-active substance (tenside) or substance mixture, aromatic substances, adjuvants and binders, for the production of a pharmaceutically effective means for the treating body parts contaminated or affected by candida albicans.

## Revendications

1. Utilisation d'une combinaison qui contient
- au moins un composé de chlorure sous forme de sel sous forme d'un sel de métal alcalin ou de métal alcalinoterreux et au moins un oxydant approprié pour la production sur place de chlore lors de la dissolution de la combinaison dans une solution aqueuse, le potentiel d'oxydation de l'oxydant dans la solution aqueuse étant au moins dans la plage de pH sollicitée plus élevé que le potentiel d'oxydation de Cl¹⁻/Cl°,
- un acide en une quantité telle que la combinaison dissoute dans une quantité déterminée de solution aqueuse produit une plage de pH < 6, de préférence < 5,5 et de manière particulièrement préférée < 5,0 ainsi
- qu'éventuellement une substance tensio-active (tenside) ou un mélange de substances, de substances aromatiques, de substances auxiliaires et de liants
pour la désinfection d'objets dentaires infestés de candida telles que les brosses à dents et les dentiers.

2. Utilisation selon la revendication 1, **caractérisée en ce que** la combinaison existe sous forme de mélange solide, en particulier de granulés ou de comprimés.

3. Utilisation selon la revendication 1 ou 2, **caractérisée en ce que** la combinaison contient des susbtances pour accélérer la dissolution (sels effervescents = effervescents), par exemple un composé qui contient du carbonate ou du bicarbonate comme par exemple du carbonate de sodium ou du bicarbonate de sodium et une quantité au moins stoechiométrique d'acide pour produire une valeur de pH < 7.

4. Utilisation selon l'une des revendications 1 à 3, **caractérisée en ce qu'**un composé de peroxosulfate d'hydrogène est contenue comme oxydant.

5. Utilisation selon l'une des revendications 1 à 4, **caractérisée en ce que** la combinaison contient au moins du monopersulfate de potassium (KHSO₅) comme oxydant, un composé de chlorure sous forme de sel, par exemple du gros sel (NaCl) comme composé de chlorure et un acide carbonique, de préférence un acide monocarboxylique, un acide dicarbonique ou tricarbonique comme un acide tartrique ou un acide citrique, pour la production d'une valeur acide de pH, une fois la combinaison dissoute.

6. Utilisation aussi selon l'une des revendications 1 à 5, **caractérisée en ce que** la combinaison contient un liant et en option des substances aromatiques, des colorants et des substances auxiliaires comme les substances pour l'adoucissement de l'eau, les matières de remplissage et équivalent et existe sous forme de comprimés ou de granulés.

7. Utilisation selon l'une des revendications 1 à 6, **caractérisée en ce que** la combinaison contient au moins un tenside (substance tensio-active ou mélange de substances).

8. Utilisation selon l'une des revendications 1 à 7, **caractérisée en ce que** la combinaison existe sous forme de comprimé nettoyant qui se dissout tout seul.

9. Utilisation selon la revendication 8, **caractérisée en ce que** la combinaison ou le comprimé nettoyant a une composition homogène.

10. Utilisation selon la revendication 8 ou 9, **caractérisée en ce que** la combinaison ou le comprimé nettoyant se dissout dans une solution aqueuse, de préférence dans de l'eau, en présence de l'objet ou de la partie du corps à désinfecter.

11. Utilisation selon l'une des revendications 1 à 10, **caractérisée en ce que** le liant est un copolymère d'oxyde d'éthylène et d'oxyde de propylène, de polyvinyle de pyrrolidone ou un copolymère de polyvinyle de pyrrolidone et d'acétate de vinyle.

12. Utilisation d'une combinaison qui contient
- au moins un composé de chlorure sous forme de sel sous forme d'un sel de métal alcalin ou de métal alcalinoterreux et au moins un oxydant approprié pour la production sur place de chlore lors de la dissolution de la combinaison dans une solution aqueuse, le potentiel d'oxydation de l'oxydant dans la solution aqueuse étant au moins dans la plage de pH sollicitée plus élevé que le potentiel d'oxydation de Cl¹⁻/Cl°,
- un acide en une quantité telle que la combinaison dissoute dans une quantité déterminée de solution aqueuse produit une plage de pH < 6, de préférence < 5,5 et de manière particulièrement préférée < 5,0 ainsi
- qu'éventuellement une substance tensio-active (tenside) ou un mélange de substances, de substances aromatiques, de substances auxiliaires et de liants
pour la production d'une substance efficace sur le plan pharmaceutique pour le traitement de parties du corps infestées ou affectées par candida albicans.
